Europäisches Patentamt

European Patent Office

Office européen des brevets

(19)

(11) Veröffentlichungsnummer: **0 003 545**
**B1**

(12) # EUROPÄISCHE PATENTSCHRIFT

(21) Anmeldenummer: **79100249.6**

(22) Anmeldetag: **29.01.79**

(51) Int. Cl.³: **A 61 K 7/28, A 23 L 1/00, A 23 G 3/00, A 23 K 1/165**

(54) **Zahnschonende, wasserarme, zuckerhaltige, speziell saccharosehaltige Lebensmittel und zahnschonende, wasserarme, pharmazeutische Zubereitungen, sowie Verfahren zu deren Herstellung**

(30) Priorität: **31.01.78 DE 2804093**

(43) Veröffentlichungstag der Anmeldung:
**22.08.79 Patentblatt 79/17**

(45) Bekanntmachung des Hinweises auf die Patenterteilung:
**29.10.80 Patentblatt 80/22**

(84) Benannte Vertragsstaaten:
**BE FR IT LU**

(56) Entgegenhaltungen:
**DE - A - 2 027 019**
**FR - A - 2 064 147**
**FR - A - 2 104 349**
**GB - A - 1 272 135**
**CHEMICAL ABSTRACTS, Vol. 69, 1968**
**Columbus, Ohio, USA,**
**DRAUS et al.: "Salivary enzymes and calculus formation", Seite 8714**

(73) Patentinhaber: **Ferrero oHG**
**D - 3570 Stadtallendorf (DE)**

(72) Erfinder: **Lembke, Andreas, Prof. Dr. Dr.**
**Institut für Virusforschung u. experiment. Medizin**
**D - 2420 Eutin-Sielbeck (DE)**
**Gorny, Dietrich**
**Wiener Straße 75**
**D - 6000 Frankfurt/Main (DE)**

(74) Vertreter: **Eitle, Werner, Dipl. Ing. et al**
**Arabellastrasse 4**
**D - 8000 München 81 (DE)**

**Zahnschonende, wasserarme, zuckerhaltige, speziell saccharosehaltige Lebersmittel und zahn-schonende, wasserarme, pharmazeutische Zubereitungen, sowie Verfahren zu deren Herstellung**

Die Erfindung betrifft zahnschonende, wasserarme, zuckerhaltige, speziell saccharosehaltige Lebensmittel und zahnsohonende, wasserarme, pharmazeutische Zubereitungen, insbesondere Zahn- und Mundpflegemittel, sowie Verfahren zu deren Herstellung.

Es ist bekannt, dass es im Mundraum in Gegenwart von Mikroorganismen durch Nahrungs-, Genuss- und Arzneimittel mit einem hohen Gehalt an Zucker, speziell Saccharose, zur Ausbildung sogenannter "Plaques" kommt und saure Abbauprodukte entstehen, die den Zahnschmelz stark angreifen. Diese Einwirkung ist unter dem Namen Karies bekannt. Man versteht darunter einen zunächst oberflächlichen Befall der Zähne, welche dann fortscheitend unter Bildung von Kavitäten zerstört werden.

Für die Enstehung der Zahnkaries sind in erster Linie diejenigen Mikroorganismen von Bedeutung, welche sich in bzw. unter den als Plaques bezeichneten Zahnbelägen finden. Die in und unter den Zahn-Plaques vorhandenen Mikroorganismen leben wie alle anderen in der Mundhöhle vorhandenen Keime von den dort nach der Nahrungsaufnahme verbleibenden Speiseresten. Besonders willkommen sind den Bakterien niedermolekulare Kohlehydrate, vor allem verschiedene Zuckerarten, weil diese die besten Energiequellen für ihre intensiven Wachstums- und Stoffwechselvorgänge darstellen.

Der Mechanismus der Kariesbildung scheint noch nicht in allen Einzelheiten exakt geklärt. Die kariogenen Mikroben, z.B. Streptococcusmutans, bilden das Ferment Dextransaccharase aus, welches den Rohrzucker (=Saccharose) in Anhydroglucose und Fructose zerlegt. Die Anhydroglucose kann sich dann zu polymeren Molekülen zusammenlagern, die als Dextrangele bezeichnet werden. Unter diesen weisen insbe-sondere Dextrangele mit einem Molekular-gewicht von 10.000 bis 200.000 eine besondere Klebkraft auf. Diese Dextrangele dienen gewissermassen als "Klebemittel", die die betreffenden Bakterien auf den Zahnoberflächen festhalten, d.h. die Plaques ausbilden. Die genannten Bakterien können dann unter den Plaques unter Luftabschluss, z.B. aus vorhandenem oder eindiffundiertem Zucker, Säuren, wie z.B. Milchsäure oder andere zahnzerstörende Säuren, erzeugen, was zur Entstehung erster Schmelzläsionen (Initialkaries) führt, d.h. es kommt zu einer Korrosion des Dentins mit den bekannten Folgeerscheinungen.

Nach Ausbildung des Plaques wird bei der hierunter erfolgenden kariösen Demineralisierung, inbesondere aus dem Schmelzapatit des Zahns unter Einwirkung von Säure allmählich Kalzium und Phosphat herausgelöst.

Bei den bisherigen Versuchen zur Hemmung von Karies sind zwei Wege eingeschlagen worden. Diese bestehen grundsätzlich entweder in der Erhöhung des Widerstandes der Zahnhartsubstanzen gegenüber dem Säureangriff oder in der Abschwächung des Säureangriffes auf den Zahn.

Unter die erstgenannte Lösung können Versuche der Trinkwasser-, Salz- oder Milchfluoridierung zusammengefasst werden, die in breitem Umfang, vor allen in den USA und der Schweiz durchgeführt worden sind. Daneben sind auch die Lokalapplikaktion von fluoridhaltigen Tabletten bzw. die Anwendung fluorierter Verbindungen, wie Natriumfluorid, Natrium-monofluorphosphat, Zinnfluorid-Kalziumpyro-phosphat oder Aminfluorid, erwähnenswert. Wenngleich nach Berichten hierdurch eine Abnahme kariöser Läsionen erzielt worden ist, so können die erzielten Ergebnisse jedoch noch nicht befriedigen. Daneben bestehen gegen die Chemikalieneinbringung, insbesondere in Trinkwasser und Nahrungsmittel, wie Milch, erhebliche Vorbehalte auch physiologischer Art. Auch reichen kollektive und individuelle Fluoridierungsmassnahmen bei der derzeitigen Ernährung der Zivilbevölkerung nicht aus, um dem kariösen Verfall der Zähne entgegenzuwirken.

Eine Kariesbekämpfung ist aber auch durch Kontrolle der Dextranbildung, beispielsweise durch Einwirkung von Chemikalien, versucht worden. So ist beispielswiese in "Journal of Dental Health", Band 22, Nr. 4, Dezember 1972, die Verwendung von Natriumoleat und -linoleat zu diesem Zweck beschrieben worden, deren Wirkung auf die Bildung von Dextran gemäss der DE—OS 2 442 825 durch wasserlösliche sekundäre Phosphate in Mitteln zur Zahnhygiene noch gesteigert werden soll. Auch ist die Verwendung von Ca-, Na- oder Mg-Salzen von Estern der Phosphorsäure mit Saccharose, Glucose oder Lactose zur Herstellung von im Mundraum kariostatisch wirksamen Zahnpflegeprodukten in der DE—AS 1 467 809 in Betracht gezogen worden. Diese Massnahmen der Blockierung des Metabolismus der Mundmikroflora, die das Dextran-Saccharose-Enzym-System zur Erzeugung der "Dental-Plaques" verwenden, haben zwar eine gewisse Verbesserung der Zahnpflegemittel, nicht jedoch die gewünschte einschneidende Bekämpfung des kariösen Zahnverfalls mit sich gebracht.

Auch die bekannten Versuche der Ausnutzung des Lyolyse-Prinzipes, nämlich der Karies entweder durch Zerstörung der kariogenen Mikroorganismen durch deren Auf- bzw. Anlösung oder durch die Auflösung bzw. Anlösung der klebenden Dextrangele vorzubeugen, konnten bislang nicht befriedigen. So ist in der DE—AS 2 011 935 die Zerstörung bzw. der Angriff kariogener Mikroben durch die Enzyme beschrieben, die aus bestimmten Streptococcus-Stämmen isoliert worden sind. Zu diesem Zweck sind drei definierte Stämme und deren Einbringung in Zahnpflegemittel angegeben worden. Daneben ist auch das Lyolyse-Prinzip auf bereits gebildetes Dextran mittels des Enzyms Dextranase gemäss der DE—OS 1 955 956 angewandt worden. Nach der AT—PS 318 815 vermag die Dextranase allerdings nur lösliches Dextran zu zersetzen, da das unlösliche Dextran einen Mutan-Anteil aufweist, der durch Dextranase nicht anngegriffen werden kann. Daher ist in dieser österreichischen Druckschrift der Einsatz von "Mutanase" vorgeschlagen worden.

Jedoch auch diese Versuche der Verbesserung von Zahnpflegemitteln durch Auflösen der Dextran erzeugenden Mikroorganismen oder der Dextrane selbst und der Inkorporierung derartiger Systeme in Zahnpflegemittel haben bislang noch nicht vollständig befriedigen können. Dies ist zum Teil darauf zurückzuführen, dass eine Pflege der Zähne nach jeder Mahlzeit, der Einnahme von Süssigkeiten etc. nicht immer möglich ist und die übliche Einwirkung der Zahnpflegemittel für sich allein nicht ausreichend ist, um die zwischenzeitlich aufgetretenen Schädigungen hintan zu halten.

Einen weiteren Weg zur Bekämpfung der Karies sah man in dem teilweisen bzw. vollständigen Ersatz von Rohrzucker durch andere, nicht-kariogene Zucker oder Zukkeralkohole bzw. durch synthetische Süssungsmittel.

Ein vollständiger Ersatz der Saccharose durch wenig bzw. nicht-kariogene Zuckeraustauschstoffe, z.B. durch die Zuckeralkohole Xylit und Sorbit, kommt aus technologischen, wirtschaftlichen, organoleptischen oder medizinischen Erwägungen bein vielen Anwendungen nicht in Frage.

Unter den synthetischen Süsstoffen besitzt Saccharin, einer der bekanntesten und am häufigsten verwendete Zuckerersatzstoffe, den Nachteil, beim Verbraucher einen unangenehmen Nachgeschmack zu erzeugen. Bei anderen Stoffen, z.B. den Cyclamaten, macht die Ärzteschaft ernsthafte Vorbehalte hinsichtlich ihrer Schädlichkeit. Die synthetischen Süsstoffe haben andererseits den Nachteil, dass sie wegen der hohnen Süsskraft einzusetzenden kleinen Mengen nicht leicht zu dosieren sind, z.B. spricht man in diesem Zusammenhang von einem "leeren Geschmack", d.h. es fehlt der erwünschte "Körper". Auch physiologische Wirkungen sind der allgemeinen Verbreitung dieser synthetischen Süsstoffe hinderlich.

Es wurde auch der Einsatz von Enzymen empfohlen. Der Zusatz von Invertase im Rahmen der Herstellung einzelner spezieller Lebensmittel ist bereits bekannt. So hat Invertase Eingang gefunden in die Kunsthonigherstellung, di Likör-, Eiscreme- und Süsswarenindustrie, wo sie zur Erzeugung weicher, stabiler Cremefüllungen oder zur Weichhaltung von Marzipan dient. Die hierbei hergestellten Endlebensmittel enthalten jedoch keinen oder zumindest keinen massgeblichen Anteil mehr an Invertase. Zudem wird in der DE—OS 1 927 411 die Verwendung von $\alpha$-Glucosidasen und/oder $\beta$-Fructosidasen zur Verhinderung der plaques-Bildung vorgeschlagen. Ausserden wurde in der DE—OS 1 948 298 zum Abbau von im Mund verbleibenden Speiseresten eine Polymer-Enzym-Verbindung beschrieben, die neutrale, alkalische, saure Proteasen neben Amylase, Lipase und Dextranase enthält.

Aus der DE—OS 2 027 019 sind Zahnpflegemittel bekannt, die wasserarm, zuckerhaltig und dadurch zahnschonend sind, dass sie Enzyme, wie Blucoseoxidase und Lactatoxidase enthalten. Entscheidend hierbei ist, dass diese Oxido-Reduktasen das Substrat mit Sauerstoff unter Bildung von Wasserstoffperoxid zersetzen. Durch dieses enzymatisch gebildete aggressive Agens werden die am karriogenen Angriff beteiligten Mikroorganismen oxidativ geschädigt bzw. zerstört. Hierdurch wird aber die Mundflora insgesamt durch an sich körperfremde medien angegriffen, was nachteilig ist.

Trotz dieser zahlreichen Versuche zur Bekämpfung der Karies, die auf die unterschiedlichsten Prinzipien aufbauen, besteht noch immer die dringende Notwendigkeit zu zusätzlichen bzw. neuartigen Massnahmen zur Verhinderung der schädlichen Folgen aus dem Säureangriff in Gegennwart kariogener Mikroorganismen, um bei der herkömmlichen Ernährung eine Zurückdrängung der Karies zu ermöglichen.

Aufgabe der vorliegenden Erfindung ist es, den schädlichen Wirkungen der Karies sowohl durch Verbesserung herkömmlicher wasserarmer Lebensmittel, die unter Verwendung von Zucker, speziell Rohrzucker, hergestellt worden sind, wie auch gegebenenfalls ergänzend durch eine gleichlaufende Verbesserung von Zahnpflegemitteln sowie pharmazeutischen Zubereitungen entgegenzuwirken. Die Verbesserung der Lebensmittel soll insbesondere dem Umstand Rechnung tragen, dass der kariogene Angriff bereits in den Zeitintervallen erfolgt, die zwischen den üblichen Zeiträumen der Zahnreinigung liegen. Insbesondere ist es erfindungsgemäss beabsichtigt. Lebensmittel ohne bzw. ohne vollständigen Ersatz der hierfür üblicherweise verwendeten Zucker, zumeist Rohrzucker, durch den Zusatz einer physiologisch unbedenklichen Substanz in "zahnschonende" Lebensmittel umzuwandeln.

Diese Aufgabe wird erfindungsgemäss dadurch gelöst, dass die vorangehend genannten wasserarmen, zuckerhaltigen, speziell saccharosehaltigen Lebensmittel und die wasserarmen, pharmazeutischen Zubereitungen, insbesondere Zahn- und Mundpflegemittel, einen Gehalt an Lactatdehydrogenase und Saccharose-invertierendem Enzym aufweisen.

Überraschenderweise hat sich bei den Versuchen gemäss der erfindung gezeigt, dass durch Zusatz von Lactatdehydrogenase (nachstehend als LDH bezeichnet) zusammen mit Saccharose-invertierendem Enzym der Kariesbefall drastisch reduziert wurde. Bei Verfütterung einer Diät, die neben einem hohen Saccharoseanteil dei beiden erfindungsgemäss vorgesehenen Enzyme in Kombination enthält, war die Anzahl der auftretenden Kariesläsionen ausserordentlich gering.

Es wird angenommen, dass durch den Zusatz von LDH in Kombination mit Saccharose-invertierendem Enzym bei wasserarmen bzw. wasserfreien Lebensmittlen bzw. Süsswaren ein Hemmsystem eingebaut wird, das bei deren Verzehr dem schädlichen Einfluss der durch Zuckerabbau entstandenen Produkte in Gegenwart kariogener Bakterien entgegenwirkt und die Lebensmittel, insbesondere Süsswaren, sowie die pharmazeutischen Zubereitungen in zahnschonende, nicht-kariogene, bzw. antikariogene umwandelt.

"Wasserarme" Lebensmittel sind solche Lebensmittel, deren Feuchtegehalt so gering ist, dass die enzymatische Reaktion nicht in Gang gesetzt wird. Vorzugsweise werden damit Lebensmittel mit einer Restfeuchte von 0,1 bis 2% verstanden.

Unter "zuckerhaltigen" Lebensmittel sind solche Lebensmittel zu verstehen, die einen Anteil natürlich vorkommender Zucker, wie Saccharose, Glucose, Fructose, etc., oder als Zuckerersatzstoffe Zuckeralkohole enthalten. Bevorzugt sind insbesondere Lebensmittel mit einem Gehalt an Saccharose. Es ist bekannt, dass Saccharose gegenüber anderen Zuckerarten bei bestimmten wasserarmen Lebensmitteln erhebliche technologische Vorteile besitzt.

Gemäss der erfindung können gegebenenfalls sogar solche Lebensmittel bzw. Süsswaren oder pharmazeutische Zubereitungen in Betracht gezogen werden, die keinen Gehalt an üblichen Zuckern aufweisen oder im wesentlichen zuckerfrei sind. Dies können Materialien sein, die auf der Grundlage von Zuckerersatzstoffen gesüsst sind, z.B. mit Saccharin, Cyclamat, aus tropishcen Pflanzen extrahierte Proteinsüsstoffe, Xylit usw. Es kann sich jedoch auch um Produkte handeln, die im wesentlichen keinen Zuckeranteil von Natur aus aufweisen und auch einer massgeblocken Süssung nicht bedürfen. Auch bei diesen Süsswaren ist die Einarbeitung von LDH zusammen mit Saccharose-invertierendem Enzym vorteilhaft. Diese können nämlich beim Verzehr des Lebensmittels mit den an den Zähnen haftenden Speiseresten anteilig im Mundraum verbleiben und dort z.B. aus dem Kohlehydratabbau resultierende kariogene Produkte unschädlich machen.

Der Ausdruck "wasserarme, zuckerhaltige Lebensmittel" soll jedoch insbesondere Süsswaren umfassen. Beispiele hierfür oder für nahestehende Produkte sind Backwaren, Desserts und Kunsthonig. Produkte von besonderem Interesse sind sogenannte Stimulantien, wie verschiedene Arten von Süssigkeiten, d.h. Hart- und Weichkaramellen (Bonbons), Fondant, Schaumzuckerwaren, Gummizuckerwaren, Lakritzwaren, Dragees, fruchtpasten, Krokant, Brausepulver, Marzipan, Persipan, Nougat, Schokoladen und Kakaoerzeugnisse, Lutscher, Pastillen, Kaugummi usw. Die Produkte von Kaugummi-Typ sind von besonderem Interesse, da solche Produkte in Langzeitberührung mit dem Speichel stehen und eine lange Verweilzeit im Mund haben. Obwohl Kaugummis normalerweise nicht verschluckt werden, werden sie jedoch als Lebensmittel angesehen und fallen somit unter der Erfindungsgedanken.

Unter Langzeitberührung in Verbindung mit längeren Verweilzeiten versteht man für die oral zu verabreichenden Produkte Zetien von wenigsten einigen Minuten.

Gemäss der erfindung kann das Lebensmittel auch ein Tierfutter sein.

Nach einer anderen Ausführungsform der Erfindung werden LDH und Saccharose-invertierendes Enzym pharmazeutischen Zubereitungen als Wirkstoff zugefügt, der natürlich auch ergänzend neben andere übliche Wirkstoffe treten kann. Als solche pharmazeutische Zubereitungen kommen vor allem Tabletten und Dragees in Frage, die neben den pharmakologisch wirkenden Substanzen Zucker enthalten. Unter Produkten, auf die die Erfindung anwendbar ist, können auch unterschiedliche Arten sogenannter quasi-medizinischer Produkte erwähnt werden, wie beispielsweise Hustenelexiere oder -sirup usw. Solche Produkte werden häufig voir dem Zubettgehen genommen, d.h. nachdem die Zähne bereits geputzt sind, und haben somit eine Langzeitwirkung auf den Zahnbelag. Eine im rahmen der Erfindung bevorzugte pharmazeutische Zubereitung stellen Mundhygienetabletten im weitesten Sinne dar, wie Zahnhygienedragees, Kautabletten bzw. zahnreinigende Kaugummis. Die vorteilhafte Wirkung des Wirkstoffes beruht auch hier darauf, dass sich aufgrund der Einspeichelung im Mundraum ein enzymatischer Schutzfilm über die Zähne legen kann, der die Umwandlung von etwa vorliegenden Zuckerresten zu kariogenen Produkten zu unterbinden vermag.

Die genannten Zahnpflegemittel können beispielsweise in Form einer Zahntablette vorliegen, die die üblichen Polier-, Binde-, Verdickungs- und Feuchthaltemittel aufweisen.

Geeignete Poliermittel, die beispeilsweise für Zahnpflegemittel verwendbar sind, sind z.B. die üblichen Kalziumphosphate, wie Trikalziumphosphat, Alkalimetalphosphate, Magnesiumcarbonat, pulverförmige Kunststoffe, wie Polymethylmetacrylat. Harnstoff-Formaldehyd-Kondensationsprodukte, etc., oder Gemische solcher Substanzen.

Daneben können die Zahnpflegemittel auch Konservierungsmittel, Aromastoffe und andere Hilfsstoffe enthalten. In Einzelfällen kann es jedoch auch erwünscht sein, durch den Zusatz von LDH und Saccharose-invertierendem Enzym die Wirkung anderer Aktivstoffe, die einen Kariesschutz aufgrund anderer Prinzipien bezwecken, zu unterstützen bzw. zu vervielfachen. Als solche üblichen Wirkstoffe können insbesondere Fluorverbindungen, wie Aminfluoride, Alkalifluoride etc., oder auch Dextranasen genannt werden.

Die erfindungsgemäss verwendete LDH ist in Handel erhältlich. Lactatdehydrogenase kann unter anderem auch as verschiedenen Mikroben, z.B. Hefe, hergestellt werden. Die aus Hefe gewonnene LDH ist verhältnismässig stabil, z.B. bleibt eine in Glyzerin gelöste Enzympräparation bei +18°C über ein Jahr lang aktiv. Daneben kann LDH auch in Pufferlösungen in einem definierten pH-Bereich ohne nennensewerten Aktivatätsverlust über längere Zeit gelagert werden. Wie Versuche im Rahmen der Erfindung gezeigt haben, erleidet die in wasserarme bzw. wasserfreie Lebensmittel oder pharmazeutische Zubereitungen eingebrachte LDH während üblicher lagerzeiten nur einen verhältnismässig geringen Aktivitätsverlust, sofern ein solcher überhaupt auftritt.

Das gemäss der Erfindung verwendete Saccharose-invertierende Enzym ist ebenfalls im Handel erhältlich. Als Saccharose-invertierende Enzyme kommen in Rahmen der Erfindung vor allem die Saccharasen in Betracht, von denen die $\alpha$-Glucosidasen und bevorzugt die ß-Fructosidasen genannt werden sollen, die Rohrzucker in Glucose und Fructose aufspalten. Es ist bekannt, dass Rohrzucker durch $\alpha$-Glucosidase gespalten wird, da er $\alpha$-glycosidisch gebundene Glycose enthält Die Saccharose ist aber auch durch ein zweites Ferment angreifbar, für deren Wirkung die Fructosehälfte des Moleküls bestimmend ist, die als ß-Fructofuranosid enthalten ist. Dieses Ferment ist daher eine ß-Fructosidase, die vielfach auch ß-h-Fructosidase genannt wird (das h soll die Furanosestruktur der Fructose bezeichnen). Diese Enzyme sind bekannt, wobei im Handel in der Regel technische Präparate aus speziellen Hefestämmen angeboten werden. Diese Enzyme weisen eine gute Lagerstabilität auf; so sind beispielsweise geriigte Trockenpräparate von Hefeinvertase mindestens 1 Jahr haltbar. Daneben kann in gewissem Umfang auch eine Lagerung in Pufferlösungen bei einem definierten pH-Bereich während relativ langer Lagerzeiten zu keinem Aktivitätsverlust führen (vgl. "Methoden der Enzymatischen Analyse", H. U. Bergmeyer, 3. Auflage 1974, Band 1, Verlag Chemie, Weinheim, Seiten 951—963). Wie im Rahmen der Erfindung durchgeführte Versuche ergaben, erleidet in wasserarme bzw. wasserfreie Lebensmittel oder pharmazeutische Zubereitungen eingebrachtes Saccharose-invertierendes Enzym während üblicher Lagerzeiten nur einen sehr geringen Aktivitätsverlust. So wurde beispeilsweise festgestellt, dass lyophilisierte ß-h-Fructosidase, die durch Einrühren in durch ansteigende Temperierung auf 45°C soweit thermoplastisch gemachte, handelsübliche Schokolade eingebracht worden war, bei dieser Einbringung lediglich einen Aktivatätsverlust von ca. 8% erfuhr, der sich bei 6-monatiger Lagerung auf nur ca. 15% erhöhte.

Erfindungsgemäss ist es bevorzugt, den Anteil an eingebrachter Enzymaktivität entspechend der anzunehmenden Lagerzeit des Materials zumindest so zu bemessen, dass dieser im Moment des Verzehrs etwa ausreichend ist, um die gewünschte Wirkung hervorzurufen.

Die Mengen an Lactatdehydrogenase und Saccharose-invertierendem Enzym, die in das Lebensmittel, die Süssware oder die pharmazeutische Zubereitung, wie Zahnreinigungstabletten, insbesondere jedoch in Pflegemittel für Zahnprothesen in Tablettenform, eingebracht werden, kann durch den Fachmann leicht aufgrund der spezifischen Enzymaktivität, des ungefähren Zuckeranteiles des Materials, sofern ein solcher gegeben ist, sowie augrund des im Bindemittel bzw. bei dessen Herstellung vorliegenden pH-Bereiche vermittelt werden.

Im allgemeinen ist eine LDH-Menge von 0,2 mg bis 500 mg pro kg geeignet. Vorzugsweise werden 0,5 mg bis 50 mg pro kg verwendet (bezogen auf die spezifische Aktivität von ca. 300 U/mg).

Ausserdem ist hier noch die Temperatur und gegebenenfalls der Wassergehalt des Materials bei Zufügung des Enzyms bzw. dessen übliche Lagerungstemperatur in Betracht zu ziehen. Der zweckmässigerweise einzubringende Gehalt der beiden Enzyme kann auch durch einfache Versuchsansätze ermittelt werden. Die Invertasemenge liegt vorzugsweise im Bereich von 0,05 g bis 5.0 g/kg (bezogen auf ca. 100 U/mg spez. Aktivität).

Bei den Ermittlungen der einzubringenden Enzymaktivität ist es z.B. nicht in jedem Fall erforderlich, den Saccharoseanteil, gegebenenfalls sogar den latenten Saccharoseanteil, der aus Vorprodukten entstehen kann, vollständig in Rechnung zu stellen. Erfahrungsgemäss wird ein relativ grosser Anteil der Nahrung verschluckt, so dass ein grosser Teil der Saccharose mit den Zähnen nicht mehr in Berührung kommt. Es genügt jedoch, wenn die auf den Zähnen abgelagerten Saccharoseanteile bezüglich der Zugabe von Enzymmengen in Rechnung gestellt werden. Durch Bürsten der Zähne und Ausspülen des Mundes mit aqua dest. wurde beispielsweise festgestellet, dass im Mittel von 50 Versuchen weniger als 1 g Rohrzucker in der Spülflüssigkeit nachweisbar war, wenn den Versuchspersonen zuvor etwa 50 g handelsübliche Schokolade verabfolgt worden war. Letztlich ist aber lediglich auf diesen im Mundraum verbleibenden bzw. zu erwartenden Restzuckeranteil abzustellen, so dass, wenn die Enzyme nicht im gleichen Anteil mit dem Nahrungsbrei mit verschluckt werden, diese gegenüber der Ausgangszuckermenge in unterproportionaler Aktivitätsmenge eingesetzt werden können. Auch für pharmazeutische Zubereitungen und insbesondere Zahnpflege- bzw. Mundhygienemittel, in die Lactatdehydrogenase und Saccharose-invertierendes Enzym eingebracht werden können, gilt, dass die einzubringenden Enzymmengen durch überschlägige Berechnungenn oder empirische Versuche ermittelt werden. In derartigen Zahnpflege- bzw. Mundhygienemittel liegt üblicherweise kein Zuckeranteil vor, weshalb es nicht erforderlich ist, die einzubringenden Enzymaktivitäten hieran zu orientieren. In diesem Falle dient die Einbringung der vorgenannten Enzyme dem Zweck, im Mundraum und insbesondere auf den Zähnen einen Flüssigkeitsfilm zu erzeugen, der karieshemmend ist, so dass eventuell verbleibende, auch durch Bürsten nicht entfernte Speisereste auch bei Anwesenheit kariogener Bakterien zu keiner Karies führen können.

Gemäss der Erfindung kann das Verhältnis der eingebrachten Enzymmenge an LDH zu der Menge an Saccharose-invertierendem Enzym in einem weiten Bereich variieren.

Die Menge an Saccharose-invertierendem Enzym sollte ausreichend sein, um eine Invertierung des Saccharosegehaltes vorzunehmen. Die hierbei rechnerisch oder durch einfache Versuchsansätze ermittelten Mengen liegen in der Regel erheblich höher als diejenigen Mengenanteile, die sich für den gleichzeitigen Einsatz von LDH als vorteilhaft erwiesen haben.

Erfindungsgemäss kann es auch vorgesehen sein, die Lactatdehydrogenase-Wirkung durch Zugabe eines geeigneten Wasserstoffakzeptors, z.B. von Nicotinamidadenindinucleotid, zu verstärken.

Die Einbringung des Enzyms LDH zusammen mit Saccharose-invertierendem Enzym wird zweckmässigerweise derart durchgeführt, dass sich eine homogene Verteilung der Enzyme innerhalb des Lebensmittels ergibt. Andereseits kann es auch bevorzugt sein, insbesondere dann, wenn das Lebensmittel selbst keine isotrope Zuckerverteilung aufweist, ebenfalls eine ungleichmässige Verteilung der eingebrachten Enzyme vorzusehen. So wäre es beispielsweise bei Schokolade, die mit ganzen Nüssen durchsetzt ist, nicht erforderlich, den Nussanteil der Schokolade mit Enzymen ebenfalls homogen zu durchsetzen.

Die Einbrinung von LDH, sowie Saccharose-invertierendem Enzym erfolgt üblicherweise bei einem pH-Wert, bei dem diese Enzyme stabil sind, sowie bei einer Temperatur, bei der die Aktivität der Enzyme nicht beeinträchtigt wird. Ein bevorzugter pH-Bereich liegt zwischen pH 4 bis 7, besonders vorteilhaft ist ein Bereich von 4,7 bis 5,2. Insbesondere bevorzugt ist es, die beiden Enzyme bei dem pH-Bereich in das Lebensmittel etc. einzubringen, der zwischen den Stabilitätsoptima der enzyme liegt. Geeignete Einbringungstemperaturen liegen zwischen 0 bis 50°C, wobei die Temperaturen zwischen 20 bis 50°C als besonders günstig anzusehen sind. Je nach Anwendungszweck kann der Temperaturbereich von 20 bis 40°C besonders bevorzugt sein.

Je nach Art des Mediums kann es wünschenswert sein, die Enzyme entweder im Verlauf des Herstellungsverfahrens oder nach Fertigstellung des Lebensmittels oder der pharmazeutischen Zubereitungen zuzusetzen, wenn beispielsweise kein massgeblicher Wasseranteil mehr vorliegt. Bei Süsswaren, wie Tafelschokolade, Pralinen etc., ist es gemäss der Erfindung bevorzugt, das Enzym erst zu einem relativ späten Zeitpunkt zuzufügen, so dass sich keine Aktivitätsverluste im Verlauf der Produktion der Süsswaren ergeben.

Im folgenden werden Versuche beschrieben, die die antikariogene Wirkung bei Zusatz von LDH in Kombination mit Saccharose-invertierendem Enzym zeigen.

Als kariogenes Substrat wurde ein handelsüblicher wasserarmer, fett- und zuckerhaltiger Brotaufstrich verwendet. Als Versuchstiere dienten weisse, homozygote Ratten vom Typ "Wistar". Der genannte Brotaufstrich wurde in 50% der Gesamtmenge einem Standard-Trockenfutter (Herilan-RM 20), aus einer vitaminreichen Protein-Fett-Diät, die unter Berücksichtigung aller Stoffwechselbedürfnisse der Ratte entwickelt worden ist, beigemischt (Herilan-RM 20 ist ein Warenzeichen).

Die lufttrockene Versuchsdiät wurde proportioniert und so dosiert, dass die Tiere während der Fütterungsperiode von 280 Tagen weiterhin an Gewicht zunehmen konnten. Die Tiere wurden in Kunststoffkäfigen gehalten, die jeweils mit Tränk- und mit Fütterungsautomaten ausgerüstet waren. Dem Trinkwasser wurde in Abständen von 30 Tagen jeweils 50 bis 60 × 10³ Streptococcus mutans-Keime pro Milliliter hinzugesetzt. Als Einstreu diente sterilisiertes Holz-Feingranulat, das in Abständen von 36 Stunden ausgewechselt wurde. Die Raumluft war klimatisiert.

Es wurden versuchsgruppen gebildet, die aus je 60 Tieren bestanden. Alle Tiere wurden täglich inspiziert und in Abständen von 30 Tagen gewogen, wobei sich keinerlei Nebenwirkungen durch den Gehalt an LDH und Saccharose-invertierendem Enzym ermitteln liessen.

Die Feststellung des kariösen Befundes, insbesondere der kariösen Läsionen, erfolgte mittels Stereomikroskop bei 12-facher Vergrösserung.

Folgende Abstufungen wurden notiert:

0=keine Karies
1=1 bis 5 Läsionen
2=5 bis 10 Läsionen
3=mehr als 10 Läsionen

Geprüft wurden folgende Diätzusammensetzungen:

A=50% Trockenfutter + 50% Brotaufstrich B=50% Trockenfutter + 50% Brotaufstrich + LDH und Saccharose-invertierendes Enzym (handelsübliche ß-h-Fructosidase)

Die LDH betrug 0,5 mg, die Menge an Saccharose-invertierendem Enzym 0,3 g pro kg Brotaufstrich.

Bei Verabreichung der Diät A traten zahlreiche kariöse Läsionen auf: Von den Tieren wurden 30 (50%) betroffen. In der nachfolgenden Tabelle sind die Versuchsergebnisse hinsichtlich der Anzahl von Läsionen als auch des jeweiligen Schweregrades zusammengefasst. Wie die Ergebnisse zeigen, wurden bei Verfütterung der Diät A zum Teil sehr starke Läsionen (>10) bei den Versuchsieren festgestellt.

Durch den Zusatz von LDH in Kombination mit Saccharose-invertierendem Enzym bei der Diät B wurde sowohl die Zahl als auch der Schweregrad der Läsionen drastisch reduziert. Die Ergebnisse zeigen deutlich die zahnschonende, nicht-kariogene Wirkung der mit Lactatdehydrogenase und Saccharose-invertierendem Enzym versehenen Diät.

Tabelle

| Versuchsgruppe | Karies-Läsionen (Zahl x Grad) |
|---|---|
| A (Diät A) | 15 x 1; 9 x 2; 6 x 3 |
| B (Diät B) | 4 x 1 |

Im folgenden werden weitere Beispiele für Lebensmittel bzw. pharmazeutische Zubereitungen gegeben, die LDH und Saccharose-invertierendes Enzym enthalten.

1. *Milchschokoladentafel*

| enthaltend | |
|---|---|
| Kakaomasse | 26 g |
| Saccharose | 60 g |
| Milchfett | 3.2 g |
| fettfreie Trockenmasse | 9,5 g |
| LDH | ca. 5—10 mg |
| ß-h-Fructosidase, die vor dem Erkalten der fertigen Schokolade bei ca. 40—45°C in homogener Verteilung eingerührt worden ist | ca. 20—25 mg |

2. *Süssungstabletten*

| enthaltend | |
|---|---|
| Xylit | 5 g |
| Saccharin-Natriumsalz | 0,7 g |
| Natriumcyclamat | 0,3 g |
| LDH (Handelsprodukt) | 0,1—0,5 mg |
| Invertase (gefriergetrocknetes Handelsprodukt) | 30 mg |

Obige Trockenmischung kann bei Gebrauch auch durch Wasserzusatz in die Form einer niedrigkalorischen antikariogenen Süssungslösung übergeführt werden.

3. *Zahneinigungsdragee*

| enthaltend | Gew.% |
|---|---|
| Magnesiumcarbonat | 10,0 |
| Siliziumdioxid | 20,0 |
| Dikalziumphosphat | 55,0 |
| Harnstoff-Formaldehyd-Kondensat | 5,50 |
| Aromastoffe | 2.0 |
| Traganth | 1,5 |
| Natriumlaurylsulfoacetat | 2,5 |
| langkettiges Aminfluorid | 2,0 |
| LDH | 0,1 |
| ß-h-Fructosidase | 0,3 |

4. *Antikariogener Kaugummi*

In eine übliche Kaugummigrundmasse (1 kg; 35 Teile Cumaronharz, 40 Teile Gummi, 10 Teile Paraffinwachs, 15 Teile trockener Latex, 67 Teile Sorbitpulver, 0,1 Teile Natriumcyclamat, 1 Teil Aroma) wurden zusätzlich 0,002 Teile antimikrobiell wirksames Natriumhexametaphosphat, 10 mg handelsübliche LDH, sowie 50 mg Invertase (gefriergetrocknetes Handelsprodukt, Hersteller Serva, Heidelberg) eingearbeitet, die Mass durchgemischt und nach dem Auswalzen in Streifen geschnitten.

In der beiliegenden Figur wird die gemäss der Erfindung insbesondere vorgesehene Tafelschokolade wiedergegeben.

**Patentansprüch**

1. Zahnschonende, wasserarme, zuckerhaltige, speziell saccharosehaltige Lebensmittel und zahnschonende, wasserarme, pharmazeutische Zubereitungen, insbesondere Zahn-und Mundpflegemittel, gekennzeichnet durch einen gehalt an Lactatdehydrogenase und Saccharose-invertierendem Enzym.

2. Zahnschonende Lebensmittel und pharmazeutische Zubereitungen nach Anspruch 1, dadurch gekennzeichnet, dass sie zur Verstärkung der Lactatdehydrogenase-Wirkung einen geeigneten Wasserstoffakzeptor enthalten.

3. Verfahren zur Herstellung zahnschonender, wasserarmer, zuckerhaltiger, speziell saccharosehaltiger Lebensmittel oder wasserarmer, pharmazeutischer Zubereitungen nach einem oder mehreren der Ansprüche 1 bis 2, dadurch gekennzeichnet, dass man dem Lebensmittel oder der pharmazeutischen Zubereitung das Enzym Lactatdehydrogenase und Saccharose-invertierendes Enzym bei oder nach Herstellung des Lebensmittels oder der pharmazeutischen Zubereitung zusetzt.

4. Verfahren nach Anspruch 3, dadurch gekennzeichnet, dass man das Enzym Lactatdehydrogenase und das Saccharose-invertierende Enzym in homogener Verteilung einbringt.

5. Verfahren nach einem der Ansprüche 3 bis 4, dadurch gekennzeichnet, dass die pharmazeutische Zubereitung oder das Lebensmittel zum Zeitpunkt des Zusatzes des Enzymes bei dem pH-Wert 4 bis 7 gehalten wird.

6. Verfahren nach einem der Ansprüche 3 bis 5, dadurch gekennzeichnet, dass die pharmazeutische Zubereitung oder das Lebensmittel zum Zeitpunkt des Zusatzes der Enzyme bei dem pH-Wert 4,7 bis 5,2 gehalten wird.

7. Verfahren nach einem der Ansprüche 3 bis 6, dadurch gekennzeichnet, dass man während der Enzymzugabe eine Temperatur von 20 bis 50°C einstellt.

8. Verfahren nach einem der Ansprüche 3 bis 7, dadurch gekennzeichnet, dass man als Lebensmittel wasserarme bzw,. -freie Süsswaren verwendet.

9. Verfahren nach einem der Ansprüche 3 bis 7, dadurch gekennzeichnet, dass das Lebensmittel ein Tierfutter darstellt.

10. Verfahren nach einem der Ansprüche 3 bis 8, dadurch gekennzeichnet, dass man als pharmazeutische Zubereitungen Tabletten, Dragees oder Mundpflegemittel verwendet.

11. Verfahren nach einem der Ansprüche 3 bis 10, dadurch gekennzeichnet, dass man als Saccharose-invertierendes Enzym ß-Fructosidase verwendet.

12. Verfahren nach einem der Ansprüche 3 bis 10, dadurch gekennzeichnet, dass man als Saccharose-invertierendes Enzym $\alpha$-Glucosidase verwendet.

13. Verfahren nach einem der Ansprüche 3 bis 12, dadurch gekennzeichnet, dass zur Verstärkung der Lactatdehydrogenase-Wirkung ein geeigneter Wasserstoffakzeptor zugegeben wird.

**Revendications**

1. Aliments sans danger pour les dents, á faible teneur en eau, contenant du sucre, spécialement du saccharose, et préparations pharmaceutiques sans danger pour les dents à faible teneur en eau, notamment produits d'hygiène dentaire et buccale, caractérisés par le fait qu'ils contiennent de la lactate déshydrogénase et une enzyme provoquant l'inversion du du saccharose.

2. Aliments et préparations pharmaceutiques sans danger pour les dents selon la revendication1, caractérisés par le fait qu'ils contiennent un accepteur d'hydrogène approprié pour renforcer l'action de la lactate déshydrogénase.

3. Procédé de préparation d'aliments sans danger pour les dents à faible teneur en eau, contenant du sucre, spécialement du saccharose, ou de compositions pharmaceutiques à faible teneur en eau selon la revendication 1 ou 2, caractérisé par le fait qu'on ajoute à l'aliment ou à la composition pharmaceutique l'enzyme lactate déshydrogénase et une enzyme provoquant l'inversion du saccharose pendant ou après la préparation de l'aliment ou de la composition pharmaceutique.

4. Procédé selon la revendication 3, caractérisé par le fait qu'on incorpore l'enzyme lactate déshydrogénase et l'enzyme provoquant l'inversion du saccharose de façon à ce qu'elles soient réparties de façon homogène.

5. Procédé selon l'une des revendications 3 ou 4, caractérisé par le fait qu'au moment de l'addition des enzymes, la valeur du pH de la composition pharmaceutique ou de l'aliment est maintenue à 4 à 7.

6. Procédé selon l'une quelconque des revendications 3 à 5, caractérisé par le fait qu'au moment de l'addition des enzymes, la valeur du pH de la composition pharmaceutique ou de l'aliment est maintenue à 4,7 à 5,2.

7. Procédé selon l'une quelconque des revendications 3 à 6, caractérisé par le fait que pendant l'addition des enzymes, on maintient une température de 20 à 50°C.

8. Procédé selon l'une quelconque des revendications 3 à 7, caractérisé par le fait qu'on utilise à titre d'aliments des sucreries à faible teneur en eau ou exemptes d'eau.

9. Procédé selon l'une quelconque des revendications 3 à 7, caractérisé par le fait que l'aliment constitue un aliment pour animaux.

10. Procédé selon l'une quelconque des revendications 3 à 8, caractérisé par le fait qu'on utilise en tant que compositions pharmaceutiques des comprimés, des dragées ou des produits d'hygiène buccale.

11. Procédé selon l'une quelconque des revendications 3 à 10, caractérisé par le fait qu'on utilise en tant qu'enzyme provoquant l'inversion du saccharose la β-fructosidase.

12. Procédé selon l une quelconque des revendications 3 à 10, caractérisé par le fait qu'on utilise en tant qu'enzyme provoquant l'inversion du saccharose, l'α-glucosidase.

13. Procédé selon l'une quelconque des revendications 3 à 12, caractérisé par le fait que pour augmenter l'activité de la lactate déshydrogénase, on ajoute un accepteur d'hydrogène approprié.

## Claims

1. Dental protective, low humidity, sugar-containing, specially saccharose-containing foodstuffs and dental protective low humidity pharmaceutical preparations, especially toothpastes and oral hygiene means, characterized by a content of lactate dehydrogenase and saccharose-inverting enzyme.

2. Dental protective foodstuffs and pharmaceutical preparations according to claim 1, characterized in that they contain a suitable hydrogen acceptor to reinforce the lactate dehydrogenase effect.

3. Process for the preparation of dental protective, low humidity, sugar-containing, specially saccharose-containing foodstuffs or low humidity, pharmaceutical preparations according to one or more of claims 1 to 2, characterized in that to said foodstuff or said pharmaceutical preparation the enzyme lactate dehydrogenase and a saccharose inverting enzyme is added either during or after the preparation of said foodstuff or pharmaceutical preparation.

4. Process according to claim 3 characterized in that the enzyme lactate dehydrogenase and the saccharose-inverting enzyme are inserted in homogenous distribution.

5. Process according to one of claims 3 to 4, characterized in that the pharmaceutical preparation or the foodstuff is kept at a pH value of 4 to 7 during the addition of the enzyme.

6. Process according to one of claims 3 to 5, characterized in that the pharmaceutical preparation or the foodstuff is kept at the time of addition of the enzyme at a pH value of 4.7 to 5.2.

7. Process according to one of claims 3 to 6, characterized in that during addition of the enzyme, a temperature of 20 to 50°C is maintained.

8. Process according to one of claims 3 to 7, characterized in that low humidity or anhydrous sweet goods are used as the foodstuff.

9. Process according to one of claims 3 to 7, characterized in that the foodstuff is an animal food.

10. Process according to one of claims 3 to 8, characterized in that tablets, pills or oral hygiene products are used as pharmaceutical preparations.

11. Process according to one of the claims 3 to 10, characterized in that as the saccharose-inverting enzyme, β-fructosidase is used.

12. Process according to one of claims 3 to 10, characterized in that as the saccharose-inverting enzyme, α-glucosidase is used.

13. Process according to one of claims 3 to 12, characterized in that to reinforce the lactate dehydrogenase effect, a suitable hydrogen acceptor is added.